Europäisches Patentamt

European Patent Office

Office européen des brevets

(11) Publication number: 0 568 460 B1

# EUROPEAN PATENT SPECIFICATION

(45) Date of publication of patent specification: 20.09.95

(51) Int. Cl.⁶: **G01N 21/31**, G01N 21/35, G01N 33/46, B27B 31/06

(21) Application number: 93401123.0

(22) Date of filing: 29.04.93

(54) **Method and apparatus for non-contact and rapid identification of wood species.**

(30) Priority: **29.04.92 US 875480**

(43) Date of publication of application:
**03.11.93 Bulletin 93/44**

(45) Publication of the grant of the patent:
**20.09.95 Bulletin 95/38**

(84) Designated Contracting States:
**DE FR GB SE**

(56) References cited:
EP-A- 0 137 696    EP-A- 0 239 408
WO-A-85/00657    WO-A-85/03351
DE-A- 4 033 485    US-A- 3 120 861
US-A- 3 890 509    US-A- 4 052 615
US-A- 4 306 151

(73) Proprietor: **MPB TECHNOLOGIES Inc.
1725 North Service Road,
Trans-Canada Highway
Dorval,
Ouebec, H9P 1J1 (CA)**

(72) Inventor: **Jamroz, Wes R.
4891 Grand Blvd.
Montreal, Ouebec H3X 3 S1 (CA)**
Inventor: **Trembley, Julien
50 Bois Soleil,
Pincourt. Ouebec J7Z 8S1 (CA)**
Inventor: **Wong, Brian
4675 Mariette Ave.
Montreal, Ouebec H4B 2 G3 (CA)**

(74) Representative: **Derambure, Christian
Cabinet Bouju Derambure (Bugnion) S.A.,
52, rue de Monceau
F-75008 Paris (FR)**

EP 0 568 460 B1

Rank Xerox (UK) Business Services
(3.10/3.09/3.3.3)

## Description

This invention concerns a method and apparatus for in-line identification of various wood species. The common method or species identification is based on visual analysis of grain and fibre morphology of samples. More precise identification may require chemical and microscopic analysis or mass spectroscopy measurements. These methods either are limited to stationary measurements or they require rather sophisticated instrumentation. In general, they are not practical for in-line applications where virtually instantaneous species identification are required. WO 85/00657 discloses a method and apparatus for identification of timber quality and structure in which a plurality of optoelectrical sensor organs make measurements using reflected light.

In accordance with this invention, a method and apparatus for non-contact and rapid species identification have been devised. The method and apparatus are derived from the fact that absorption of infra-red radiation by wood depends on type of species which is being irradiated. It has been experimentally demonstrated, that a relationship exists between the types of wood being irradiated with intense infra-red radiation and the density of surface modifications caused by this radiation. Furthermore, it has been determined, that the density of surface modifications can be translated into a quantitative measure by using an optical beam. An analyzed wood sample is irradiated with infra-red beams two (2) times: first with lower- and then with higher-energy density beam. The effects of these irradiations are analyzed with scanning optical beams. Intensities of the scattered optical beams are recorded by optical detector(s). It has been found, that various wood species may be identified by experimentally determining the following two (2) parameters:

$$SIP_1 = ( I_{in} - I_1 ) / I_{in} \qquad [1]$$
$$SIP_2 = ( I_{in} - I_2 ) / I_{in} \qquad [2]$$

where:

$SIP_1$ - Species Identification Parameter. The values of the parameter $SIP_1$ are predetermined experimentally for a preselected energy density $P_1$ of the infra-red radiation. The values of the $SIP_1$ are predetermined for all the range of the moisture content for given species;

$SIP_2$ - Species Identification Parameter. The values of the parameter $SIP_2$ are predetermined experimentally for a preselected energy density $P_2$ (such that $P_2 > P_1$) of the infra-red radiation.

The values of the $SIP_2$ are predetermined for all the range of the moisture content for given species;

$I_{in}$ - is the intensity of optical radiation scattered from the analyzed portion of a substance surface prior to irradiation with the infra-red radiation.

$I_1$ - is the intensity of optical radiation scattered from the analyzed portion of a substance surface after it was irradiated with the infra-red radiation of the lower intensity ($P_1$);

$I_2$ - is the intensity of optical radiation scattered from the analyzed portion of a substance surface after it was irradiated with the infra-red radiation of the higher intensity (P2);

It has been found that a set of the parameters $SIP_1$ and $SIP_2$ is the characteristic property of every type of wood and it can be used as the basis for the identification of various species. These parameters are also function of a moisture content. Therefore, they may be simultaneously used for the determination of the moisture content. The values of the SIPs are predetermined experimentally for all the range of moisture content of given species. These values are recorded and used as the calibration data.

The values of $P_1$ and $P_2$ are preselected energy densities of the infra-red radiation which is used in the measurements. These values are characteristic for a given apparatus. The values of $I_1$, $I_2$ and $I_{in}$ are measured by the optical detector(s) during the identification process. It has been experimentally demonstrated, that every set of SIP1 and SIP2 determines explicitly a given species and its moisture content.

The invention allows for non-contact rapid identification of various wood species according to the methods of claims 1, 2, 3, 4, 9, 10 and apparatus according to claim 16. The identification of species can be done either for moving or stationary samples. The additional advantage of the method is that the apparatus can be placed at a distance from the analyzed substance because all the measurements may be carried out by low-divergence beams.

Figures 1 and 2 illustrate schematic views of two versions of the apparatus. Figures 3 - 8 illustrate data obtained by using exemplary apparatus for species identification in accordance with this invention.

FIGURE 1 illustrates a schematic view of apparatus capable of identification of stationary samples. The apparatus includes an energy density adjustable infra-red source, a single source of optical radiation, and an optical detector.

FIGURE 2 illustrates a schematic view of apparatus capable of identification of moving samples. The apparatus includes an energy density adjustable source of infra-red radiation, two sources of optical radiation, and two optical detectors.

FIGURE 3 presents a graph plotting the species identification parameters $SIP_1$ and $SIP_2$ for lumber samples of spruce and balsam.

FIGURE 4 presents a graph plotting the species identification parameters $SIP_1$ and $SIP_2$ for lumber samples of pine and balsam.

FIGURE 5 presents a graph plotting the species identification parameters $SIP_1$ and $SIP_2$ for lumber samples of douglas-fir and balsam.

Figure 6 presents a graph plotting the species identification parameters $SIP_1$ and $SIP_2$ versus moisture content for lumber samples of spruce and balsam.

Figure 7 presents a graph plotting the species identification parameters $SIP_1$ and $SIP_2$ versus moisture content for lumber samples of pine and balsam.

Figure 8 presents a graph plotting the species identification parameters $SIP_1$ and $SIP_2$ versus moisture content for lumber samples of douglas-fir and balsam.

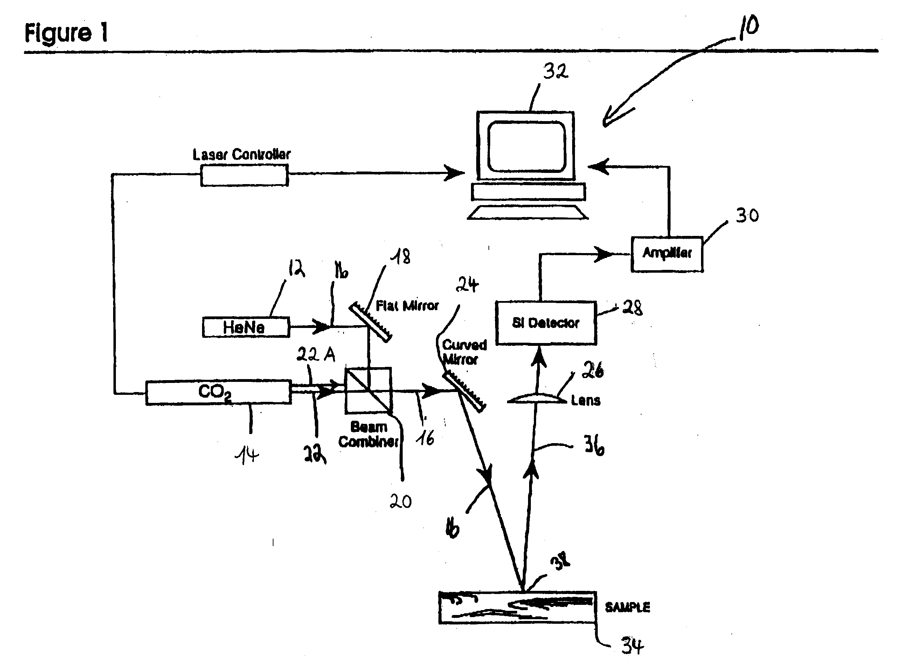

The apparatus of the present invention is suittable for the species identification of stationary or moving samples. In the case of stationary samples, the sample is first scanned with an analyzing optical light source such as HeNe laser. The scattered optical radiation ($I_1n$) is collected by lens and recorded by an optical detector. This is followed by irradiating the sample surface with a first energy density ($P_1$) low-divergence light beam, for example an infra-red beam, from $CO_2$ laser. The $CO_2$ beam is combined with the HeNe laser beam by using a ZnSe beam combiner. The $CO_2$ laser beam is focused on the sample by a curved mirror. The following step is to scan the same sample surface again with the optical light source and to record the intensity of scattered optical radiations ($I_1$) by the optical detector. The next step is to irradiate the sample with a second energy density ($P_1$) low-divergence light beam from the $CO_2$ laser ($P_2 > P_1$) and to scan the surface again with the HeNe beam. The intensity ($I_2$) of scattered radiation is recorded by the detector. The arrangement of optical axes of the detector and the radiation sources is such that they converge at the sample surface. All the system components are controlled by a computer. The computer is equipped with a data aquisition board, which is used for data recording and analysis. Referring specifically to Figure 1, the apparatus, generally designated by reference numeral 1∅, comprises an optical light source 12 such as a HeNe laser and a low-divergence light beam source 14 providing intense infra-red radiation. The optical light emitted from optical light source 12 is represented by arrow 16. Flat mirror 18 directs optical light 16 to beam combiner 2∅. The intense infra-red radiation emitted by low-divergence light beam source 14 is represented by arrow 22. Curved mirror 24 reflects both optical light 16 and intense infra-red radiation 22 after their passage through beam combiner 2∅. Lens 26 is provided to converge reflected light 36 to optical detector 28. Amplifier 3∅ amplifies the signal provided from detector 28. The components of the system are controlled by computer 32 which is equipped with a data acquisition board which is used for data recording and analysis. The arrangement of optical axis of the detector and the radiation sources is such that they converge at the sample surface.

When it is desired to identify the species of stationary sample 34, a selected area 38 of sample 34 is first scanned with optical beam 16 from HeNe laser 12 which has been reflected through mirrors 18 and 24 and passed through beam combiner 2∅. The reflected optical radiation, which is identified by arrow 36, is collected by lens 26, forwarded to optical detector 28 through amplifier 3∅ and recorded by computer 32. Selected area 38 of sample 34 is then irradiated by a first intense infra-red radiation 22 from low-divergence light beam source 14 such as a $CO_2$ laser. The intense infra-red radiation 22 is combined to optical light 16 through beam combiner 2∅, preferably a ZnSe beam combiner. The intense infra-red radiation 22 is focused on selected area 38 of sample 34 through mirror 24. This scanning causes the microstructure of sample 34 to be permanently modified at selected area 38. Following irradiation, selected surface 38 of sample 34 is scanned again with optical light 16 and the reflected intensity 36 is recorded by the optical detector 28.

Selected area 38 of sample 34 is then irradiated by a second intense infra-red radiation 22A also from low-divergence light beam source. It should be noted that for illustration purposes, infra-red radiation 22A is shown above infra-red radiation 22, even though radiation 22A passes through the same axis as radiation 22. Infra-red 22A is also combined to optical light 16 through beam combiner 2∅. Following this second irradiation, selected surface 38 of sample 34 is scanned once more with optical light 16 and the reflected intensity 36 is recorded by optical detector 29. The analyzed wood species are identified by calculating parameters $SIP_1$ and $SIP_2$ according to formulas [1,2] and then comparing their values with predetermined and recorded calibration data.

In the case of a moving sample, a conveyor causes the sample to move past infra-red radiation from a $CO_2$ laser and optical beams from two HeNe laser. The scattered optical radiation ($I_{in}$, $I_1$ and $I_2$) are recorded by two optical detectors. The arrangement of optical axes of the radiation sources and the detectors is such, that they are in line with respect to the direction in which the sample is being moved. The infra-red $CO_2$ laser irradiates the surface with two (2) pulses of an intense radiation of predetermined energy densities $P_1$ and $P_2$ (such that $P_2 > P_1$). This radiation causes the surface modifications. The modified portion of the surface is then scanned with the optical beam. The optical radiation scattered from the modified surface is recorded by the detector. All the system components are controlled by a computer. The computer is equipped with a data aquisition board, which is used for data recording and analysis.

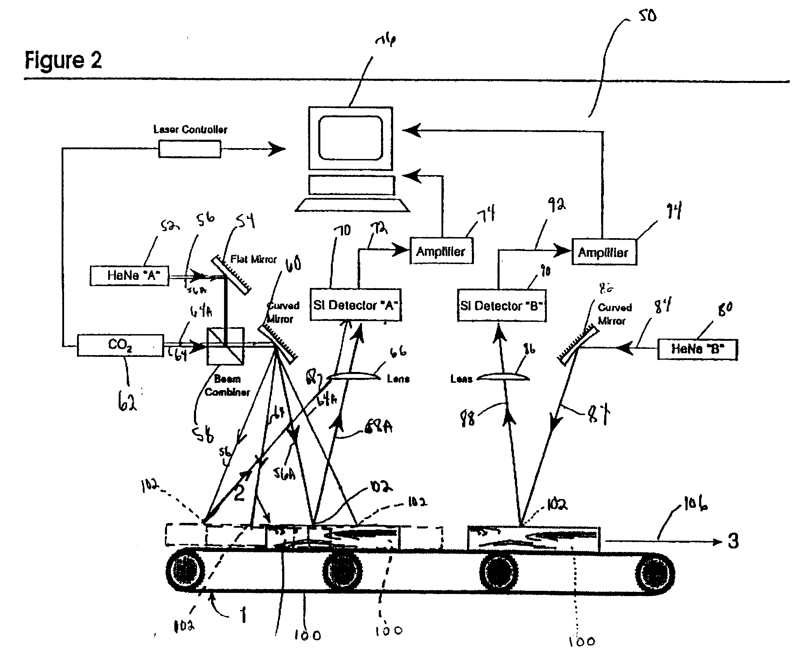

Referring specifically to Figure 2, the apparatus, generally designated by reference numeral 50, presents some similarities with the apparatus described in Figure 1. Hence, the apparatus 50 comprises a first optical light source 52, a flat mirror 54 to reflect optical light 56 to beam combiner 58 and a curved mirror 60 to focus optical light beam 56 and 56A. Also included is a low-divergence light beam source 62 which provides intense infra-red radiation 64 and 64A. Lens 66 provides reflected optical light 68 to detector 70. The detected signal 72 is amplified through amplifier 74 and analyzed by computer 76.

The system 50 illustrated in Figure 2 also comprises a further optical light source 80 which is also preferably a HeNe laser, a second curved mirror 82 to focus optical light 84 and a second lens 86 to provide reflected optical light 88 to detector 90. The detected signal 92 is amplified by amplifier 94 and analyzed by computer 76.

When it is desired to identify the species of sample 100 from selected area 102, sample 100 is moved on conveyor 104 and selected area 102 is subjected to optical light 56. Reflected optical light 68 is analyzed through detector 70. The sample 100 which continuously moves on conveyor 104 is then irradiated at the same selected area 102 by a first intense infra-red radiation 64 which causes a permanent modification of the microstructure of sample 100 at selected area 102. Sample 100 is then scanned with optical light 56A at selected surface 102 and reflected light 68A is recorded by detector 70.

Selected area 102 of sample 100 is then irradiated by a second intense infra-red radiation 64A. Sample 100 then continues its path in the direction of arrow 106 and is scanned again at selected area 102 by optical beam 84 from further light source 80. Reflected optical light 88 is then analyzed through second detector 90. The arrangement of optical axis of the radiation sources and detectors is such that they are in line with respect to the

direction 106 in which sample 100 is being moved.

The sample is identified by comparing the calculated values of the parameters $SIP_1$ and $SIP_2$ with predetermined and recorded data.

In the following examples, a $CO_2$ laser has been used as the source of infra-red radiation (MPB's model IN-70 wavelength 10.6 micron, 60 Watt output power, 7 mm beam diameter, pulsed at 50 msec). HeNe lasers have been used as the sources of the optical beams (Melles Griot's model 05LHP 991$_2$ wavelength 0.6328 micron, 10 m Watt output power, 0.7 mm beam diameter). The curved mirror (1 meter radius of curvature, 2.54 cm (1 inch) diameter) was used to focus the laser beam on the samples. The distance between the sample surface and the mirror was approximately 0.5 meter. The optical detectors used were manufactured by EG&G (Model 30809). The computer was equipped with a data acquisition board (a 12 bit analog-to-digital converter).

It has been determined, that there is a linear relationship between the optimum intensity of the $CO_2$ laser beam and the speed of the conveyor. For example, the 60 Watt output power corresponds to the optimum speed of the conveyor of 60.8 cm/s (120 feet per minute). Also, it has been determined, that the optimum value of higher intensity infra-red radiation (i.e. $P_2$) should be two (2) times higher than the $P_2$.

**Example 1**

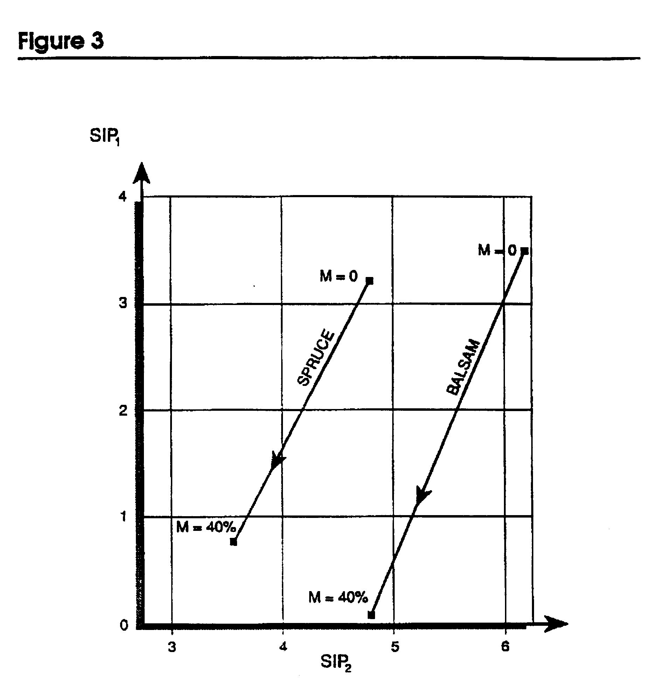

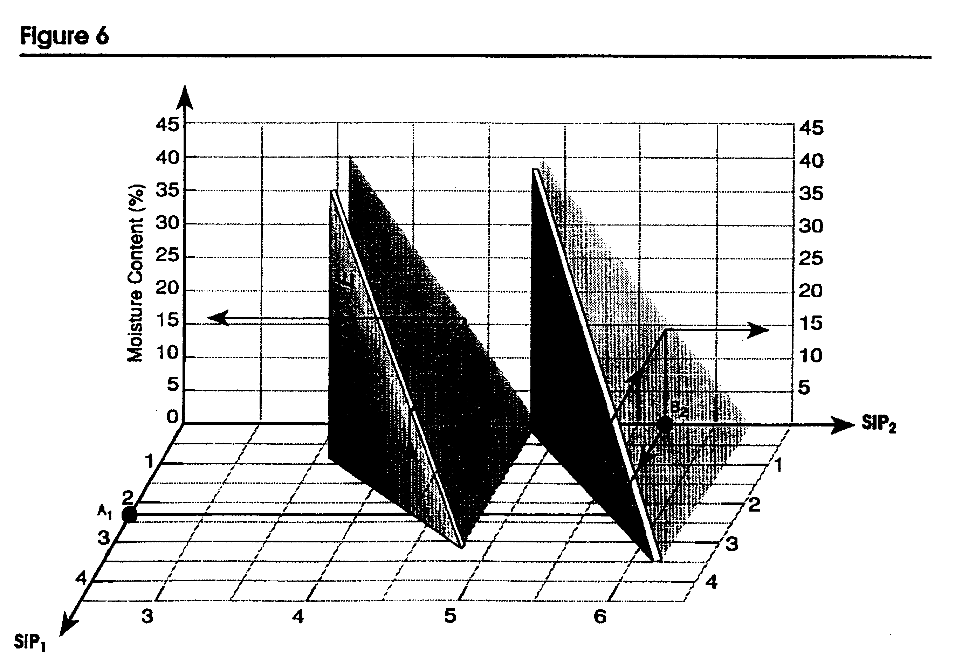

Pieces of two by four lumber spruce and balsam were analyzed with the described apparatus. All the analyzed samples contained various moisture content in the range from 0% to 40%. The samples were moving with the speed of 7.6 cm/s (15 feet/minute). A $CO_2$ laser was used as the source of the infra-red radiation. The preselected values of the infra-red energy densities were : $I_1$ = 0.02 and $I_2$ = 0.04 [Joule/mm$^2$]. The graph of the species identification parameters $SIP_1$ and $SIP_2$ are plotted on Figure 3. All the points along each of the plotted lines correspond to various moisture content for a given species. The arrows indicate moisture content gradient from 0% to 40%. Figure 6 is a 3-dimensional representationh of the species identification and moisture measurement calibration data for spruce and balsam.

The arrows on the Figure 6 show the data interpretation sequence. For example, if the measured value of $SIP_1$ is 2.3 (i.e. point $A_1$), then there are two (2) possible corresponding values of $SIP_2$ i.e. $B_1$ and $B_2$. If the measured value of $SIP_2$ is in the range of 4.3, then it is explicit that the analyzed sample is spruce and its moisture content is in the range of 16%. If the measured value of $SIP_2$ is in the range of 5.7, then the analyzed sample in balsam and its moisture content is 14%.

## Example 2

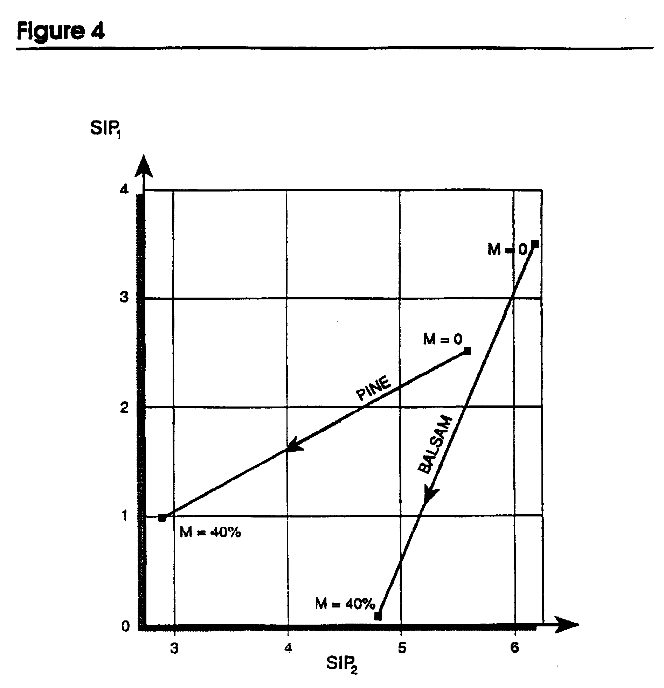

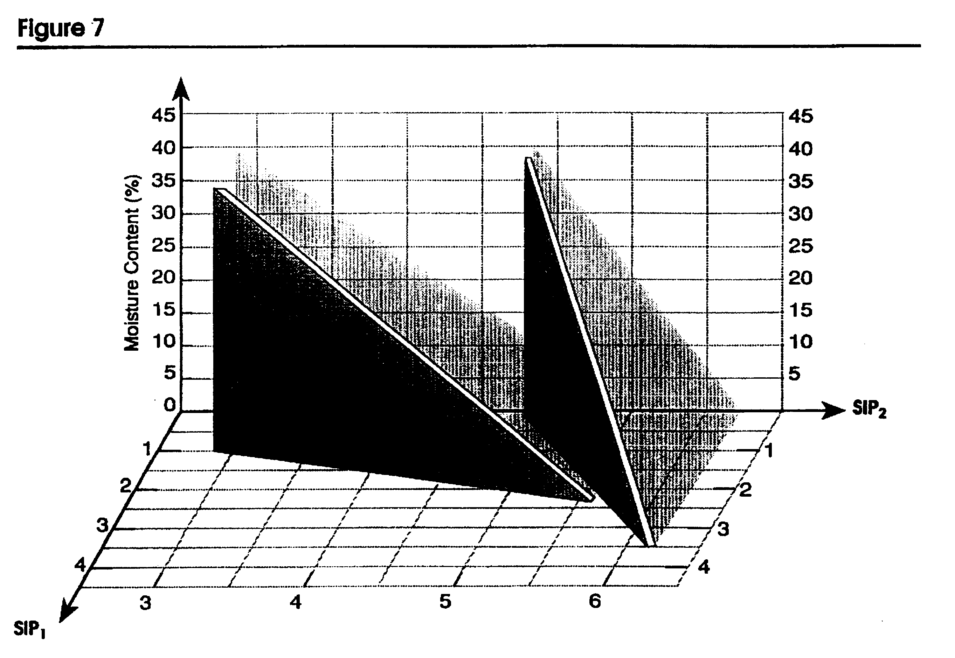

Pieces of two by four lumber pine and balsam were analyzed with the described apparatus. All the analyzed samples contained various moisture content in the range from 0% to 40%. The samples were moving with the speed of 7.6 cm/s (15 feet/minute). A $CO_2$ laser was used as the source of the infra-red radiation. The preselected values of the infra-red energy densities were : $I_1 = 0.02$ and $I_2 = 0.04$ [Joule/mm²]. The graph of the species identification parameters $SIP_1$ and $SIP_2$ are plotted on Figure 4. All the points along each of the plotted lines correspond to various moisture content for a given species. The arrows indicate moisture content gradient from 0% to 40%. Figure 7 is a 3-dimensional representationh of the species identification and moisture measurement calibration data for pine and balsam.

## Example 3

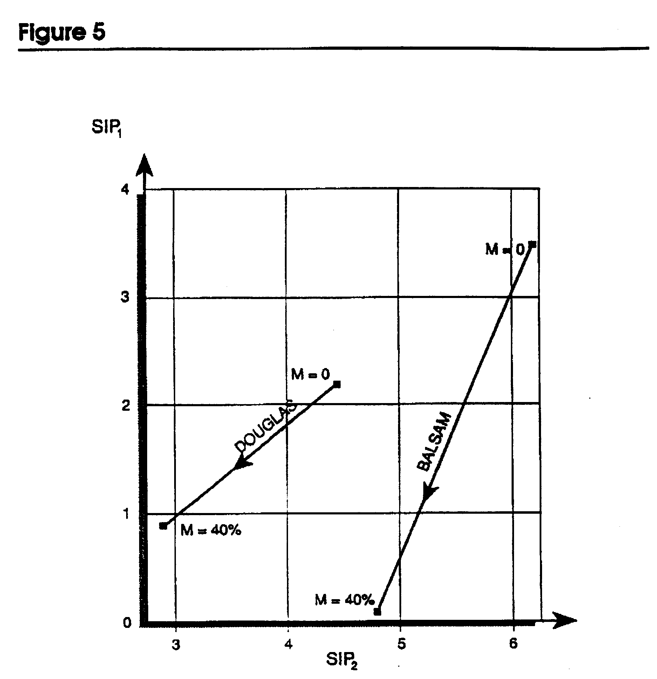

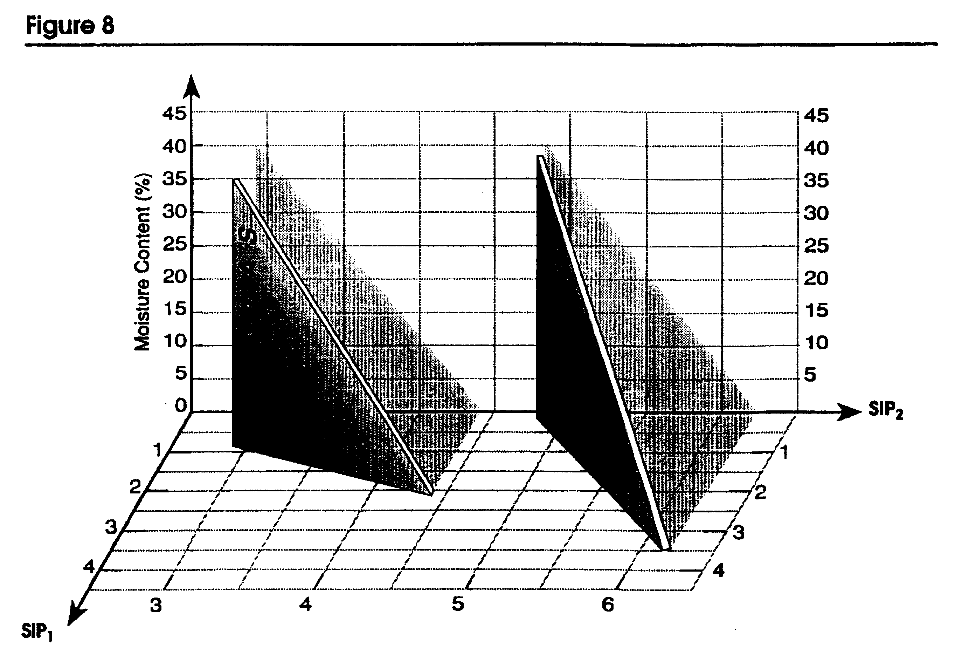

Pieces of two by four lumber douglas-fir and balsam were analyzed with the described apparatus. All the analyzed samples contained various moisture content in the range from 0% to 40%. The samples were moving with the speed of 7.6 cm/s (15 feet/minute). A $CO_2$ laser was used as the source of the infra-red radiation. The preselected values or the infra-red energy densities were : $I_1 = 0.02$ and $I_1 = 0.04$ [Joule/mm²]. The graph of the species identification parameters $SIP_1$ and $SIP_2$ are plotted on Figure 5. All the points along each of the plotted lines correspond to various moisture content for a given species. The arrows indicate moisture content gradient from 0% to 40% . Figure 8 is a 3-dimensional representationh of the species identification and moisture measurement calibration data for douglas-fir and balsam.

## Claims

1. A method for the non-contact identification of wood species, said method comprising:
   - subjecting a wood sample to be identified to an optical light beam of predetermined energy density and measuring the intensity of said optical light reflected from said sample;
   - irradiating an area of said wood sample with a laser beam of first and second predetermined energy density levels, each of said density levels being sufficient to produce modifications of the microstructure of said sample;
   - subjecting said irradiated area to an optical light beam of predetermined energy density and measuring the intensity of said optical light reflected from said irradiated area after irradiation at each of said first and second energy density levels; and
   - identifying said wood species by comparing said measurements of optical light reflected from said sample and from said irradiated area to predetermined wood species optical light reflected data.

2. A method for the non-contact identification of wood species, said method comprising:
   - subjecting a wood sample to be identified to an optical light beam of predetermined energy density and measuring the intensity of said optical light reflected from said sample;
   - irradiating at least one area or said wood sample with laser beams of first and second predetermined energy density levels, each of said density levels being sufficient to produce modifications of the microstructure of said sample at one or more areas thereof;
   - subjecting said irradiated areas to an optical light beam of predetermined energy density and measuring the intensity of said optical light reflected from said irradiated areas after irradiation at each of said first and second energy density levels; and
   - identifying said wood species by comparing said measurements of optical light reflected from said sample and from said irradiated areas to predetermined wood species optical light reflected data.

3. A method for non-contact and rapid identification of wood species comprising:
   (a) measuring initial intensity of scattered optical radiation of predetermined energy density from a selected area on surface of analyzed wood sample;
   (b) subjecting selected area on surface of said wood sample to modifications of its microstructure by irradiating said selected area on surface of said wood sample with infra-red radiant energy at two (2) or more predetermined energy density levels;
   (c) measuring the intensities of scattered optical radiation from said modified area on surface of said wood sample after irradiation at each of said first and second energy density levels, and;

(d) identifying the type of wood species by using said intensities of scattered optical radiation from said modified area of said wood sample and comparing them with predetermined data for the analyzed types of wood species.

4. A method of identifying the type of wood species by making measurements at different locations therealong, comprising :

(a) measuring initial intensities of scattared optical radiation of predetermined energy density from preselected areas along on surface of analyzed wood sample;

(b) subjecting each one of plurality of preselected areas along on surface of said wood sample to modifications of its microstructure by irradiating said selected areas on surface of said wood sample with infrared radiant energy at two (2) or more predetermined energy density levels;

(c) measuring the intensities of scattered optical radiation from said modified areas on surface of said wood sample after irradiation at each of said first and second energy density levels, and;

(d) identifying the type of species by using said measurements of intensities of scattered optical radiation from said modified areas of said wood sample and comparing them with predetermined data for the analyzed types of wood species.

5. The method as claimed in claim 4, wherein for each surface areas, steps (a), (b), (c) and (d) as set forth in claim 2 are carried out simultaneously.

6. The method as claimed in claim 3, wherein said piece of wood moves at a speed relative to radiant energy sources.

7. The method as claimed in claim 4, wherein said piece of wood moves at a speed relative to radiant energy sources.

8. The method as claimed in claim 5, wherein said piece of wood moves at a speed relative to radiant energy sources.

9. A method for non-contact and rapid identification of wood species comprising:

(a) measuring initial intensity of scattered optical radiation of predetermined energy density from a selected area on surface of analyzed wood sample;

(b) subjecting selected area on surface of said wood sample to modifications of its microstructure by irradiating said selected area on surface of said wood sample with infra-red radiant energy at two (2) or more predetermined energy density levels;

(c) measuring the intensities of scattered optical radiation from said modified area on surface of said wood sample after irradiation at each of said first and second energy density levels, and;

(d) identifying the type of species by using said intensities of scattered optical radiation from said modified area of said wood example and comparing them with predetermined data for the analyzed types of wood species.

10. A method of identifying the type of wood species by making measurements at different locations therealong, comprising:

(a) measuring initial intensities of scattered optical radiation of predetermined energy density from preselected areas along a surface of an analyzed wood sample;

(b) subjecting each one of plurality of preselected areas along on surface of said wood sample to modifications of its microstructure by irradiating said selected areas on surface of said wood sample with infrared radiant energy at two (2) or more predetermined energy density levels;

(c) measuring the intensities of scattered optical radiation from said modified areas on surface of said wood sample after irradiation at each of said first and second energy density levels, and;

(d) identifying the type of species by using said intensities of scattered optical radiation from said modified areas of said wood sample and comparing them with predetermined data for the analyzed types of wood species.

11. The method as claimed in claim 1Ø, wherein for each surface areas, steps (a), (b), (c) and (d) an set forth in claim 8 are carried out simultaneously.

12. The method as claimed in claim 9, wherein said piece of wood moves at a speed relative to radiant energy sources.

13. The method as claimed in claim 10, wherein said piece of wood moves at a speed relative to radiant energy sources.

14. The method as claimed in claim 11, wherein said piece of wood moves at a speed relative to radiant energy sources.

**15.** An apparatus for carrying out the method of any one of claims 1 to 14.

**16.** An apparatus for the non-contact identification of wood species samples, said apparatus comprising in combination :
- an optical light source for subjecting said wood species sample to optical light;
- an optical detector positioned relative to said optical light source to measure the intensity of optical light reflected by said wood species samples after having subjected said wood species samples to optical light; and
- an infra-red source to irradiate said wood species samples on a selected area of said samples at an energy density sufficient to modify the microstructure of said wood species samples,

means for actuating said optical light source and detector and means for controlling said infra-red source, to measure the intensity or optical light reflected by said wood species samples after having subjected said samples to said optical light, and to measure the intensity of optical light reflected by said selected area of said wood species samples after having irradiated said area with said infra-red source at a plurality of energy densities and means for identifying said wood species by comparing said optical light reflected from said sample and from said modified area to predetermined wood species optical reflected data.

**Patentansprüche**

**1.** Verfahren zum berührungslosen Identifizieren von Holzarten, wobei das Verfahren umfaßt:
- Einwirkenlassen eines Strahls optischen Lichtes vorgegebener Energiedichte auf eine zu identifizierende Holzprobe und Messen der Intensität des von der Probe reflektierten Strahls optischen Lichtes;
- Bestrahlen eines Bereiches der Holzprobe mit einem Laserstrahl eines ersten und eines Zweiten vorgegebenen Energiedichtepegels, wobei jeder der Dichtepegel ausreicht, um Modifizierungen der Mikrostruktur der Probe zu bewirken;
- Einwirkenlassen eines Strahls optischen Lichtes vorgegebener Energiedichte auf den bestrahlten Bereich und Messen der Intensität des von dem bestrahlten Bereich nach der Bestrahlung bei dem ersten und dem zweiten Energiedichtepegel reflektierten optischen Lichtes und

- Identifizieren der Holzarten durch des Vergleichen der Messungen des von der Probe und von dem bestrahlten Bereich reflektierten optischen Lichtes mit vorgegebenen Reflexionswerten optischen Lichtes von Holzarten.

**2.** Verfahren zum berührungslosen Identifizieren von Holzarten, wobei das Verfahren umfaßt:
- Einwirkenlassen eines Strahls optischen Lichtes vorgegebener Energiedichte auf eine zu identifizierende Holzprobe und Messen der Intensität des von der Probe reflektierten optischen Lichtes;
- Bestrahlen wenigstens eines Bereiches der Holzprobe mit Laserstrahlen eines ersten und eines zweiten vorgegebenen Energiedichtepegels, wobei jeder der Dichtepegel ausreicht, um Modifizierungen der Mikrostruktur der Probe in einem oder mehreren Bereichen derselben Zu bewirken;
- Einwirkenlassen eines Strahls optischen Lichtes mit vorgegebener Energiedichte auf die bestrahlten Bereiche und Messen der Intensität des von den bestrahlten Bereichen nach der Bestrahlung bei dem ersten und dem zweiten Energiedichtepegel reflektierten optischen Lichtes und
- Identifizieren der Holzarten durch Vergleichen der Messungen des von der Probe und von den bestrahlten Bereichen reflektierten optischen Lichtes mit vorgegebenen Reflexionswerten optischen Lichtes von Holzarten.

**3.** Verfahren zum berührungslosen und schnellen Identifizieren von Holzarten, das umfaßt:
(a) Messen der Anf angsintensität von einem ausgewählten Bereich an einer Oberfläche einer analysierten Holzprobe gestreuter optischer Strahlung vorgegebener Energiedichte;
(b) Erzeugen von Modifizierungen der Mikrostruktur des ausgewählten Bereiches auf der Oberfläche der Holzprobe durch Bestrahlen des ausgewählten Bereiches an der Oberfläche der Holzprobe mit Infrarotstrahlungsenergie mit zwei (2) oder mehr vorgegebenen Energiedichtepegeln;
(c) Messen der Intensitäten von dem modifizierten Bereich an der Oberfläche der Holzprobe nach dem Bestrahlen mit dem ersten und dem zweiten Energiedichtepegel gestreuter optischer Strahlung und
(d) Identifizieren des Typs von Holzarten unter Verwendung der Intensitäten von dem modifizierten Bereich der Holzprobe gestreuter optischer Strahlung und Vergleichen derselben mit vorgegebenen Werten für die analysierten Typen von Holzarten.

4. Verfahren zum Identifizieren des Typs von Holzarten durch Ausführen von Messungen an verschiedenen Stellen an selbigen, das umfaßt:

(a) Messen von Anfangsintensitäten von ausgewählten Bereichen an der Oberfläche einer analysierten Holzprobe gestreuter optischer Strahlung vorgegebener Energiedichte;

(b) Erzeugen von Modifizierungen der Mikrostruktur jedes der Vielzahl ausgewählter Bereiche an der Oberfläche der Holzprobe durch Bestrahlen der ausgewählten Bereiche an der Oberfläche der Holzprobe mit Infrarotstrahlungsenergie mit zwei (2) oder mehr vorgegebenen Energiedichtepegeln;

(c) Messen der Intensitäten von den modifizierten Bereichen an der Oberfläche der Holzprobe nach Bestrahlen mit dem ersten und dem zweiten Energiedichtepegel gestreuter optischer Strahlung und

(d) Identifizieren des Typs von Arten unter Verwendung der Messungen der Intensitäten von den modifizierten Bereichen der Holzprobe gestreuter optischer Strahlung und Vergleichen derselben mit vorgegebenen Werten für die analysierten Typen von Holzarten.

5. Verfahren nach Anspruch 4, wobei für jeden Oberflächenbereich die in Anspruch 2 aufgeführten Schritte (a), (b), (c) und (d) gleichzeitig ausgeführt werden.

6. Verfahren nach Anspruch 3, wobei sich das Stück Holz mit einer Geschwindigkeit in bezug auf Strahlungsenergiequellen bewegt.

7. Verfahren nach Anspruch 4, wobei sich das Stück Holz mit einer Geschwindigkeit in bezug auf Strahlungsenergiequellen bewegt.

8. Verfahren nach Anspruch 5, wobei sich das Stück Holz mit einer Geschwindigkeit in bezug auf Strahlungsenergiequellen bewegt.

9. Verfahren zum berührungslosen Identifizieren von Holzarten, das umfaßt:

(a) Messen der Anfangsintensität von einem ausgewählten Bereich an der Oberfäche einer analysierten Holzprobe gestreuter optischer Strahlung vorgegebener Energiedichte;

(b) Erzeugen von Modifizierungen der Mikrostruktur des ausgewählten Bereiches an der Oberfläche der Holzprobe durch Bestrahlen des ausgewählten Bereiches an der Oberfläche der Holzprobe mit Infrarotstrahlungsenergie mit zwei (2) oder mehr vorgegebenen Energiedichtepegeln;

(c) Messen der Intensitäten von dem modifizierten Bereich an der Oberfläche der Holzprobe nach Bestrahlen mit dem ersten und dem zweiten Energiedichtepegel gestreuter optischer Strahlung und

(d) Identifizieren des Typs von Arten unter Verwendung der Intensitäten von dem modifizierten Bereich der Holzprobe gestreuter optischer Strahlung und Vergleichen derselben mit vorgegebenen Werten für die analysierten Typen von Holzarten.

10. Verfahren zum Identifizieren des Typs von Holzarten durch Ausführen von Messungen an verschiedenen Stellen an selbigen, das umfaßt:

(a) Messen von Anfangsintensitäten von ausgewählten Bereichen an einer Oberfläche einer analysierten Holzprobe gestreuter optischer Strahlung vorgegebener Energiedichte;

(b) Erzeugen von Modifizierungen der Mikrostruktur jedes der Vielzahl ausgewählter Bereiche an der Oberfläche der Holzprobe durch Bestrahlen der ausgewählten Bereiche an der Oberfläche der Holzprobe mit Infrarotstrahlungsenergie mit zwei (2) oder mehr vorgegebenen Energiedichtepegeln;

(c) Messen der Intensitäten von den modifizierten Bereichen an der Oberfläche der Holzprobe nach Bestrahlen mit dem ersten und dem zweiten Energiedichtepegel gestreuter optischer Strahlung und

(d) Identifizieren des Typs von Arten unter Verwendung der Intensitäten von den modifizierten Bereichen der Holzprobe gestreuter optischer Strahlung und Vergleichen derselben mit vorgegebenen Werten für die analysierten Typen von Holzarten.

11. Verfahren nach Anspruch 10, wobei für jeden Oberflächenbereich die in Anspruch 8 aufgeführten Schritte (a), (b), (c) und (d) gleichzeitig ausgeführt werden.

12. Verfahren nach Anspruch 9, wobei sich das Stück Holz mit einer Geschwindigkeit in bezug auf Strahlungsenergiequellen bewegt.

13. Verfahren nach Anspruch 10, wobei sich das Stück Holz mit einer Geschwindigkeit in bezug auf Strahlungsenergiequellen bewegt.

14. Verfahren nach Anspruch 11, wobei sich das Stück Holz mit einer Geschwindigkeit in bezug auf Strahlungsenergiequellen bewegt.

15. Vorrichtung zum Ausführen des Verfahrens nach einem der Ansprüche 1 bis 14.

16. Vorrichtung zum berührungslosen Identifizieren von Holzartenproben, wobei die Vorrichtung in Kombination umfaßt:
- eine Quelle optischen Lichtes, mit der die Holzartenprobe optischem Licht ausgesetzt wird;
- einen optischen Detektor, der in bezug auf die Quelle optischen Lichtes so angeordnet ist, daß er die Intensität von den Holzartenproben reflektierten optischen Lichtes mißt, nachdem die Holzartenproben optischem Licht ausgesetzt worden sind, und
- eine Infrarotquelle, die die Holzartenproben in einem ausgewählten Bereich der Proben mit einer Energiedichte bestrahlt, die ausreicht, um die Mikrostruktur der Holzartenproben zu modifizieren,
eine Einrichtung zum Betätigen der Quelle optischen Lichtes und des Detektors sowie eine Einrichtung zum Steuern der Infrarotquelle, um die Intensität nach dem Einwirken des optischen Lichtes auf die Proben durch die Holzarten proben reflektierten optischen Lichtes zu messen und um die Intensität von dem ausgewählten Bereich nach dem Bestrahlen des Bereiches der Holzartenprobe mit der Infrarotquelle mit einer Vielzahl von Energiedichten reflektierten optischen Lichtes zu messen, sowie eine Einrichtung zum Identifizieren der Holzarten durch Vergleichen des von der Probe und von dem modifizierten Bereich reflektierten optischen Lichtes mit vorgegebenen Reflexionswerten optischen Lichtes von Holzarten.

**Revendications**

1. Procède pour l'identification sans contact d'espèces de bois, ledit procédé comprenant :
(a) l'exposition d'un échantillon de bois à identifier à un faisceau lumineux optique de densité d'énergie prédéterminée et la mesure de l'intensité de ladite lumière optique réfléchie par ledit échantillon;
(b) l'irradiation d'une zone dudit échantillon de bois avec un faisceau laser de premier et deuxième niveaux d'énergie prédéterminés, chacun desdits niveaux de densité étant suffisant pour produire des modifications de la microstructure dudit échantillon;

(c) l'exposition de ladite zone irradiée à un faisceau lumineux optique de densité d'énergie prédéterminée et la mesure de l'intensité de ladite lumière réfléchie par ladite zone irradiée après irradiation à chacun desdits premier et deuxième niveaux de densité d'énergie; et
(d) l'identification desdites espèces de bois en comparant lesdites mesures de la lumière optique réfléchie par ledit échantillon et par ladite zone irradiée aux données prédéterminées de lumière optique réfléchie des espèces de bois.

2. Procédé pour l'identification sans contact d'espèces de bois, ledit procédé comprenant :
(a) l'exposition d'un échantillon de bois à identifier à un faisceau lumineux optique de densité d'énergie prédéterminée et la mesure de l'intensité de ladite lumière optique réfléchie par ledit échantillon;
(b) l'irradiation d'au moins une zone dudit échantillon de bois avec des faisceaux laser de premier et deuxième niveaux d'énergie prédéterminés, chacun desdits niveaux de densité étant suffisant pour produire des modifications de la microstructure dudit échantillon à une ou plusieurs de ses zones;
(c) l'exposition desdites zones irradiées à un faisceau lumineux optique de densité d'énergie prédéterminée et la mesure de l'intensité de ladite lumière réfléchie par lesdites zones irradiées après irradiation à chacun desdits premier et deuxième niveaux de densité d'énergie; et
(d) l'identification desdites espèces de bois en comparant lesdites mesures de la lumière optique réfléchie par ledit échantillon et par lesdites zones irradiées aux données prédéterminées de lumière optique réfléchie des espèces de bois.

3. Procédé pour l'identification sans contact et rapide d'espèces de bois comprenant :
(a) la mesure de l'intensité initiale du rayonnement optique diffusé de densité d'énergie prédéterminée d'une zone sélectionnée sur la surface de l'échantillon de bois analysé;
(b) l'exposition de la zone sélectionnée sur la surface dudit échantillon de bois à des modifications de sa microstructure en irradiant ladite zone sélectionnée sur la surface dudit échantillon de bois avec de l'énergie rayonnante infrarouge à deux (2) niveaux de densité d'énergie prédéterminés ou plus;
(c) la mesure des intensités du rayonnement optique diffusé de ladite zone modifiée sur la surface dudit échantillon de bois après irradiation à chacun des premier et deuxième niveaux de densité d'énergie, et

(d) l'identification du type d'espèces de bois en utilisant lesdites intensités de rayonnement optique diffusé de ladite zone modifiée dudit échantillon de bois et leur comparaison avec des données prédéterminées pour les types d'espèces de bois analysés.

4. Procédé pour identifier le type d'espèces de bois en procédant à des mesures en différents endroits, comprenant :

(a) la mesure des intensités initiales du rayonnement optique diffusé de densité d'énergie prédéterminée de zones présélectionnées sur la surface de l'échantillon de bois analysé;

(b) l'exposition de chacune des plusieurs zones présélectionnées sur la surface dudit échantillon de bois à des modifications de sa microstructure en irradiant lesdites zones sélectionnées sur la surface dudit échantillon de bois avec de l'énergie rayonnante infrarouge à deux (2) niveaux de densité d'énergie prédéterminés ou plus;

(c) la mesure des intensités du rayonnement optique diffusé desdites zones modifiées sur la surface dudit échantillon de bois après irradiation à chacun desdits premier et deuxième niveaux de densité d'énergie, et

(d) l'identification du type d'espèces en utilisant lesdites mesures d'intensité de rayonnement optique diffusé desdites zones modifiées dudit échantillon de bois et leur comparaison avec des données prédéterminées pour les types d'espèces de bois analysés.

5. Procédé selon la revendication 4, dans lequel pour chaque zone de surface, les étapes (a), (b), (c) et (d) comme décrites dans la revendication 2 sont simultanément exécutées.

6. Procédé selon la revendication 3, dans lequel ladite pièce de bois se déplace à une vitesse dépendant des sources d'énergie rayonnante.

7. Procédé selon la revendication 4, dans lequel ladite pièce de bois se déplace à une vitesse dépendant des sources d'énergie rayonnante.

8. Procédé selon la revendication 5, dans lequel ladite pièce de bois se déplace à une vitesse dépendant des sources d'énergie rayonnante.

9. Procédé pour l'identification sans contact et rapide d'espèces de bois comprenant :

(a) la mesure de l'intensité initiale du rayonnement optique diffusé de densité d'énergie prédéterminée d'une zone sélectionnée sur la surface de l'échantillon de bois analysé;

(b) l'exposition de la zone sélectionnée sur la surface dudit échantillon de bois à des modifications de sa microstructure en irradiant ladite zone sélectionnée sur la surface dudit échantillon de bois avec de l'énergie rayonnante infra-rouge à deux (2) niveaux de densité d'énergie prédéterminés ou plus;

(c) la mesure des intensités du rayonnement optique diffusé de ladite zone modifiée sur la surface dudit échantillon de bois après irradiation à chacun desdits premier et deuxième niveaux de densité d'énergie, et

(d) l'identification du type d'espèces en utilisant lesdites intensités de rayonnement optique diffusé de ladite zone modifiée dudit échantillon de bois et leur comparaison avec des données prédéterminées pour les types d'espèces de bois analysés.

10. Procédé pour identifier le type d'espèces de bois en réalisant des mesures en différents endroits, comprenant :

(a) la mesure des intensités initiales du rayonnement optique diffusé de densité d'énergie prédéterminée de zones présélectionnées sur une surface d'un échantillon de bois analysé;

(b) l'exposition de chacune des plusieurs zones présélectionnées sur la surface dudit échantillon de bois à des modifications de sa microstructure en irradiant lesdites zones sélectionnées sur la surface dudit échantillon de bois avec de l'énergie rayonnante infra-rouge à deux (2) niveaux de densité d'énergie prédéterminés ou plus;

(c) la mesure des intensités du rayonnement optique diffusé desdites zones modifiées sur la surface dudit échantillon de bois après irradiation à chacun desdits premier et deuxième niveaux de densité d'énergie, et

(d) l'identification du type d'espèces en utilisant lesdites intensités de rayonnement optique diffusé desdites zones modifiées dudit échantillon de bois et leur comparaison avec des données prédéterminées pour les types d'espèces de bois analysés.

11. Procédé selon la revendication 10, dans lequel pour chaque zone de surface, les étapes (a), (b), (c) et (d) comme décrites dans la revendication 9 sont simultanément exécutées.

12. Procédé selon la revendication 9, dans lequel ladite pièce de bois se déplace à une vitesse dépendant des sources d'énergie rayonnante.

13. Procédé selon la revendication 10, dans lequel ladite pièce de bois se déplace à une vitesse dépendant des sources d'énergie rayonnante.

14. Procédé selon la revendication 11, dans lequel ladite pièce de bois se déplace à une vitesse dépendant des sources d'énergie rayonnante.

15. Appareil pour exécuter le procédé d'une quelconque des revendications 1 à 14.

16. Appareil pour l'identification sans contact d'échantillons d'espèces de bois, ledit appareil comprenant en combinaison :

   - une source lumineuse optique pour exposer ledit échantillon d'espèce de bois à la lumière optique;
   - un détecteur optique positionné par rapport à ladite source de lumière optique pour mesurer l'intensité de la lumière optique réfléchie par lesdits échantillons d'espèces de bois après avoir exposé lesdits échantillons d'espèces de bois à la lumière optique; et
   - une source infrarouge pour irradier lesdits échantillons d'espèces de bois sur une zone sélectionnée desdits échantillons à une densité d'énergie suffisante pour modifier la microstructure desdits échantillons d'espèces de bois, des moyens pour activer ladite source de lumière optique et le détecteur, et des moyens pour contrôler ladite source infrarouge, afin de mesurer l'intensité de la lumière optique réfléchie par lesdits échantillons d'espèces de bois après avoir exposé lesdits échantillons à ladite lumière optique, et afin de mesurer l'intensité de la lumière optique réfléchie par ladite zone sélectionnée desdits échantillons d'espèces de bois après avoir irradié ladite zone avec ladite source infrarouge à une pluralité de densités d'énergie et des moyens pour identifier lesdites espèces de bois en comparant ladite lumière optique réfléchie par ledit échantillon et par ladite zone modifiée aux données réfléchies optiques des espèces de bois prédéterminées.

# Figure 1

EP 0 568 460 B1

# Figure 2

EP 0 568 460 B1

## Figure 3

# Figure 4

# Figure 5

Figure 6

Figure 7

**Figure 8**